# EUROPEAN PATENT APPLICATION

(11) **EP 4 067 880 A1**
(43) Date of publication of application: **05.10.2022**
(21) Application number: 22164598.9
(22) Date of filing: 28.03.2022
(51) Int. Cl.: G01N 21/77, G01N 21/05, G01N 21/64, B01L 3/00, G01N 27/26, G01N 33/543

(54) **MULTILAYER STRUCTURE FOR A BIOSENSOR, BIOSENSOR AND METHOD FOR ITS MANUFACTURE**

(30) Priority: 29.03.2021 FR 2103221
(71) Applicant: Linxens Holding, 78200 Mantes-la-Jolie (FR)
(72) Inventor: GERMAIN, François, 78200 Mantes-la-Jolie (FR); VASSAL, Simon, 78200 Mantes-la-Jolie (FR)
(74) Representative: Grünecker Patent- und Rechtsanwälte PartG mbB

(57) **Abstract**

The present invention concerns a multilayer structure (2, 102) for a biosensor, comprising a base layer (4, 104), a biocompatible layer (6, 106) comprising a reagent (8, 108) on the base layer (4, 104), a self-adhesive layer (10, 110) on the biocompatible layer (6, 106), such that the reagent (8, 108) is at least partially aligned with a channel (12, 112) formed in the self-adhesive layer (10, 110), and a top layer (14, 114) on the self-adhesive layer (10, 110). According to the present invention, the biocompatible layer (6, 106) is deposited directly onto the base layer (4, 104) and is adhesive. The present invention also concerns a biosensor and a method for the manufacture of such a multilayer structure.

## Description

The present invention relates to a multilayer structure for a biosensor, to a biosensor as well as to a method for its manufacture. In particular, the present invention relates to the field of optical biosensors. Optical biosensors are usually characterized in that they are devoid of electrodes.

A biosensor, also known as a biological detector, is an analytical device comprising a transducer and a reagent, i.e. a biologically active sensitive element (enzymes, cells, antibodies, etc.), which interacts with a fluid to be analysed. The biochemical response is converted into an electrical signal from which at least one parameter of the fluid may be determined.

Biosensors of this type usually comprise a multilayer structure provided with channels formed in a biocompatible and hydrophilic material, and in which the fluid to be analysed can be conveyed towards the reagent in a manner such as to generate a chemical reaction.

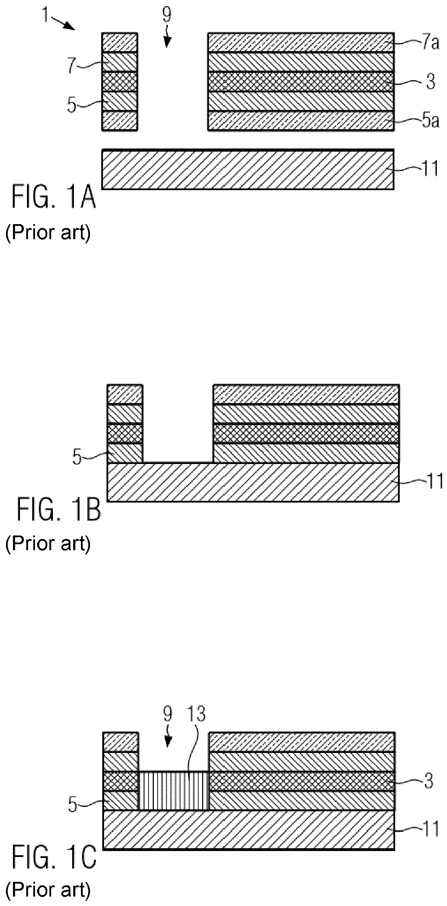

A multilayer structure for a biosensor and its manufacturing method are known in the art and are illustrated in Figures 1A to 1E.

As illustrated in Figure 1A, the known multilayer structure 1 comprises a biocompatible layer 3 sandwiched between two adhesive layers 5, 7 wherein their respective surfaces which are not in contact with the biocompatible layer are each covered with a detachable film or release film 5a, 7a. Thus, in the step of Figure 1A, the corresponding surface of the adhesive layer 5, 7 covered by the release film 5a, 7a is not sticky.

During the method for the manufacture of the known structure 1, the multilayer assembly comprising the biocompatible layer 3, the two adhesive layers 5, 7 and the two release films 5a, 7a is pierced in a manner such as to form a through opening 9.

The known structure 1 further comprises a base layer 11.

In a subsequent step of the method for the manufacture of the known structure 1, illustrated in Figure 1B, the release film 5a protecting the adhesive surface of the adhesive layer 5 is removed and the adhesive layer 5 is deposited directly on the base layer 11.

Then, as illustrated in Figure 1C, a reagent 13 is deposited directly onto the base layer 11 through the through opening 9. The reagent 13 is deposited in a manner such as to be in direct contact with the base layer 11, the adhesive layer 5 and the biocompatible layer 3.

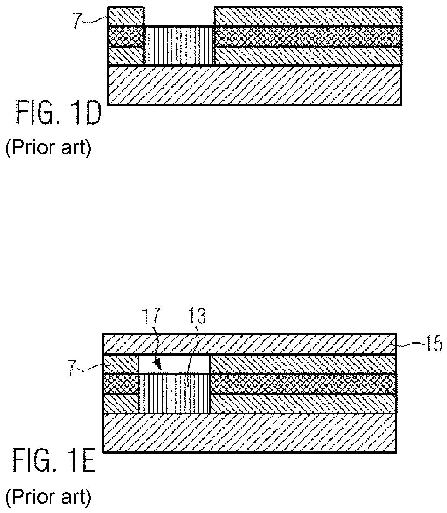

In the next step illustrated in Figure 1D, the release film 7a protecting the adhesive surface of the adhesive layer 7 is removed.

Finally, as illustrated in Figure 1E, a top layer 15 is deposited directly onto the adhesive surface of the adhesive layer 7. Thus, a channel 17 in which the fluid to be analysed can circulate is formed between the reagent 13 and the top layer 15.

This known method nevertheless requires two steps dedicated to removing each of the release films 5a, 7a. These release films 5a, 7a are, moreover, liable to be damaged by the step for perforation in order to form the through opening 9.

The objective of the present invention is to provide a multilayer structure for a biosensor which is simpler to manufacture.

The objective of the present invention is achieved by means of a multilayer structure for a biosensor, comprising: a base layer, a biocompatible layer comprising a reagent, the biocompatible layer is deposited on the base layer, a self-adhesive layer deposited on the biocompatible layer, such that the reagent is at least partially aligned with a channel formed in the self-adhesive layer, and a top layer deposited on the self-adhesive layer. In accordance with the present invention, the biocompatible layer is deposited directly onto the base layer and is adhesive.

By virtue of the adhesive properties of the biocompatible layer, the latter can be deposited directly onto the base layer without the intermediary of an additional adhesive layer, and therefore of a corresponding release film, in contrast to the known prior art structure illustrated in Figure 1E.

Thus, the multilayer structure for a biosensor in accordance with the present invention, has no electrodes, is advantageously simplified because the number of steps required to manufacture a structure of this type is reduced compared with the known structure. In particular, it is no longer necessary to provide a step for removing a second release film.

The multilayer structure for a biosensor in accordance with the present invention may be further improved by means of the following embodiments.

In accordance with one embodiment, the reagent may be an electrochemical or fluorescent substance.

When the reagent is an electrochemical substance, the biosensor is an electrochemical biosensor. The phenomenon of biological recognition is based on the formation of a complex which will induce a specific electrical signal. Electrochemical biosensors enable high sensitivity measurements to be made with a rapid response time.

When the reagent is a fluorescent substance, the biosensor is an optical biosensor. The biological recognition phenomenon is then based on a fluorescent signal. The fluorescent molecule may be a fluorophore or fluorochrome.

In accordance with one embodiment, the reagent may be deposited in a groove of the biocompatible layer.

The reagent is thus accessible to the fluid to be analysed which can move in the groove of the biocompatible layer. The reagent can thus be brought into contact with the fluid to be analysed so as to generate a chemical reaction.

In accordance with one embodiment, the width of said groove of the biocompatible layer may be smaller than the width of the channel of the self-adhesive layer.

The difference in width between the channel of the self-adhesive layer and the groove in the biocompatible layer makes it possible to avoid polluting or contaminating the reagent with the adhesive substances from a release film which would have been deposited on the pressure-sensitive adhesive layer.

In accordance with one embodiment, said groove in the layer of the biocompatible layer may be a through-groove.

Thus, the reagent can be deposited directly on the base layer through the through-groove.

In accordance with one embodiment, the self-adhesive layer may be a pressure-sensitive self-adhesive layer.

Since the self-adhesive layer is pressure-sensitive, it does not require a solvent, water or heat to activate the adhesive properties of said layer, which means that manufacture can be simplified.

In accordance with one embodiment, the self-adhesive layer, in particular the pressure-sensitive self-adhesive layer, may have hydrophilic properties in order to improve the permeability of the structure with liquids such as water, blood or urine; this is particularly advantageous in structures intended to be used in membrane immunoassays or in in vitro diagnostic and medical devices.

In accordance with one embodiment, the self-adhesive layer is an acrylic adhesive, in particular an inert acrylic adhesive which is suitable for medical applications.

In accordance with one embodiment, the thickness of the self-adhesive layer is between 10µm and 30µm. By virtue of this small thickness range for the self-adhesive layer, the structure can remain pliable and flexible.

The objective of the present invention is also achieved by a biosensor comprising a transducer and a multilayer structure as described above.

The objective of the present invention is also achieved by means of a method for the manufacture of a multilayer structure for a biosensor, comprising the steps of: providing a base layer, providing a biocompatible and adhesive layer which comprises a reagent, directly on the base layer, providing a layer which is self-adhesive and comprises a channel in which a fluid can move on or over the biocompatible layer, in a manner such that the reagent of the biocompatible layer is at least partially aligned with the channel of the self-adhesive layer, providing a top layer deposited on the self-adhesive layer.

By virtue of the adhesive properties of the biocompatible layer, the latter can be deposited directly onto the base layer without the intermediary of an adhesive layer, and therefore of a corresponding release film, in contrast to the known prior art structure illustrated in Figure 1E.

Thus, the manufacturing method in accordance with the present invention is advantageously simplified compared with the known method, because the number of steps for manufacturing the multilayer structure is reduced. In particular, it is no longer necessary to provide a step for removing a second release film. The manufacturing process is therefore made simpler, more rapid and therefore less costly.

The method for the manufacture of a multilayer structure for a biosensor according to the present invention may be further improved by means of the following embodiments.

In accordance with one embodiment, the reagent may be an electrochemical or fluorescent substance.

When the reagent is an electrochemical substance, the biosensor is an electrochemical biosensor. The phenomenon of biological recognition is based on the formation of a complex which will induce a specific electrical signal. Electrochemical biosensors enable high sensitivity measurements to be made with a rapid response time.

When the reagent is a fluorescent substance, the biosensor is an optical biosensor. The biological recognition phenomenon is then based on a fluorescent signal. The fluorescent molecule may be a fluorophore or fluorochrome.

The reagent, i.e. the electrochemical or fluorescent substance, may be deposited by piezoelectric deposition, by screen printing, by flat screen printing, by rotary screen printing, by inkjet printing or by offset printing. The reagent can therefore be deposited by means of known and controlled techniques.

According to one embodiment, the manufacturing method may comprise: a first step in which a through opening of width L1 is formed through the self-adhesive layer and a release film is deposited on a surface of the self-adhesive layer, and a second step during which the self-adhesive layer is deposited directly onto the biocompatible and adhesive layer, in particular by lamination, and a third step during which a groove of width L2 is formed by etching into the biocompatible and adhesive layer, the width L2 being smaller than the width L1, and a fourth step in which the biocompatible and adhesive layer is deposited directly onto the base layer, in particular by lamination, and a fifth step in which the reagent is deposited in the groove of width L2, and a sixth step during which the release film is removed from the self-adhesive layer, and a seventh step during which a top layer is deposited onto the self-adhesive layer, in particular by lamination.

The difference in width between the opening in the self-adhesive layer and release film and the channel in the biocompatible layer means that adhesive substances of the release film can be prevented from coming into contact with the reagent. Thus, pollution or contamination of the reagent by substances of this type is advantageously avoided.

According to one embodiment, the manufacturing method may comprise: a first step during which a release film is deposited onto a surface of the self-adhesive layer, which is in turn deposited on the biocompatible and adhesive layer which is deposited on the base layer, a second step for perforation to form a through hole through the self-adhesive layer and the release film and forming a blind hole in the biocompatible and adhesive layer, a third step during which the reagent is deposited in the blind hole of the biocompatible and adhesive layer, a fourth step during which the release film is removed from the self-adhesive layer, and a fifth step during which a top layer is deposited on the self-adhesive layer, in particular by lamination.

Since all the through holes and blind holes are formed concomitantly during the same step, a step for aligning the holes in the manufacturing method according to the present invention is therefore not necessary.

In accordance with one embodiment, the perforation in the second step may be carried out by laser or by chemical etching.

Perforation by laser or by etching enables both through holes and blind holes to be formed. For the formation of a through hole, a laser, for example of the CO₂ or UV type ("UV" stands for ultraviolet), is used at a higher power than that used for the formation of a blind hole. A lower power is in fact used for the blind hole so that the material of the layer is not completely perforated from one side to the other by the laser. The chemical etching may be carried out by means of a suitable solvent such as a solution based on concentrated sulphuric acid, or on concentrated chloric acid or concentrated sodium hydroxide or on butyl derivatives of the butyl ether type.

The invention and its advantages will now be explained in more detail below by means of preferred embodiments, in particular with reference to the accompanying figures, in which:
Figure 1A illustrates a first step of a known method for the manufacture of a multilayer structure according to the prior art.
Figure 1B illustrates a second step of a known method for the manufacture of a multilayer structure according to the prior art.
Figure 1C illustrates a third step of a known method for the manufacture of a multilayer structure according to the prior art.
Figure 1D illustrates a fourth step of a known method for the manufacture of a multilayer structure according to the prior art.
Figure 1E illustrates a prior art multilayer structure.
Figure 2 illustrates a multilayer structure in accordance with a first embodiment of the present invention.
Figure 3 illustrates a multilayer structure according to a second embodiment of the present invention.
Figure 4A illustrates a first step of a method for the manufacture of the multilayer structure in accordance with the first embodiment of the present invention.
Figure 4B illustrates a second step of a method for the manufacture of the multilayer structure in accordance with the first embodiment of the present invention.
Figure 4C illustrates a third step of a method for the manufacture of the multilayer structure according to the first embodiment of the present invention.
Figure 4D illustrates a fourth step of a method for the manufacture of the multilayer structure according to the first embodiment of the present invention.
Figure 4E illustrates a fifth step of a method for the manufacture of the multilayer structure according to the first embodiment of the present invention.
Figure 4F illustrates a sixth step of a method for manufacturing the multilayer structure according to the first embodiment of the present invention.
Figure 4G illustrates a seventh step of a method for the manufacture of the multilayer structure according to the first embodiment of the present invention.
Figure 5A illustrates a first step of a method for the manufacture of the multilayer structure in accordance with the second embodiment of the present invention.
Figure 5B illustrates a second step of a method for the manufacture of the multilayer structure according to the second embodiment of the present invention.
Figure 5C illustrates a third step of a method for the manufacture of the multilayer structure in accordance with the second embodiment of the present invention.
Figure 5D illustrates a fourth step of a method for the manufacture of the multilayer structure in accordance with the second embodiment of the present invention.
Figure 5E illustrates a fifth step of a method for the manufacture of the multilayer structure in accordance with the second embodiment of the present invention.

The invention will now be described in more detail using advantageous embodiments by way of example and with reference to the figures. The embodiments described are simply possible configurations and it should be borne in mind that the individual features as described above may be provided independently of each other or may be omitted completely when implementing the present invention.

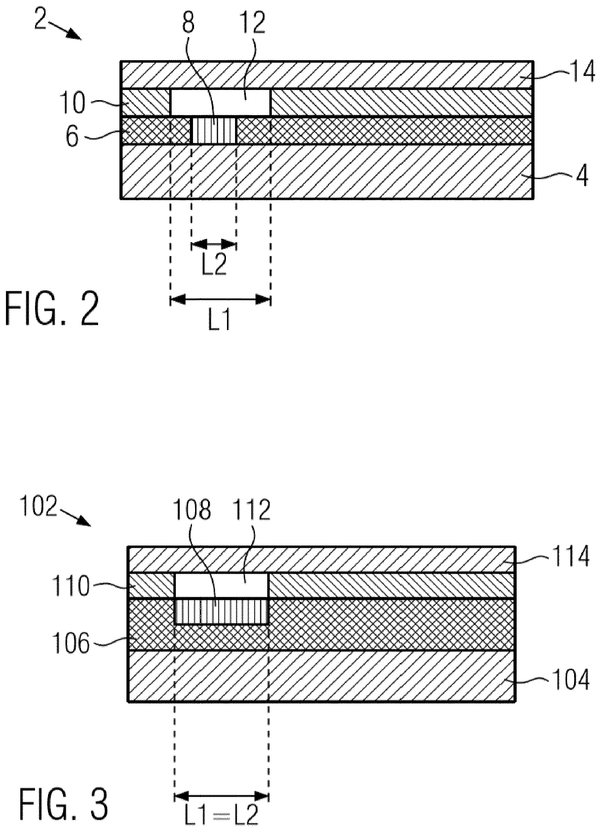

Figure 2 illustrates a multilayer structure 2 according to a first embodiment of the present invention.

The multilayer structure 2 is a structure for an optical biosensor and thus has no electrodes and/or no metal layer.

The multilayer structure 2 comprises a base layer 4 and a biocompatible layer 6, comprising a reagent 8, on the base layer 4. The reagent 8 is an electrochemical or fluorescent substance. The multilayer structure 2 further comprises a pressure-sensitive self-adhesive layer 10 on the biocompatible layer 6, so that the reagent 8 is aligned with a channel 12 of width L1 formed in the self-adhesive layer 10.

The multilayer structure 2 also comprises a top layer 14 on the self-adhesive layer 10.

In accordance with the present invention, the biocompatible layer 6 is deposited directly onto the base layer 4 and is adhesive.

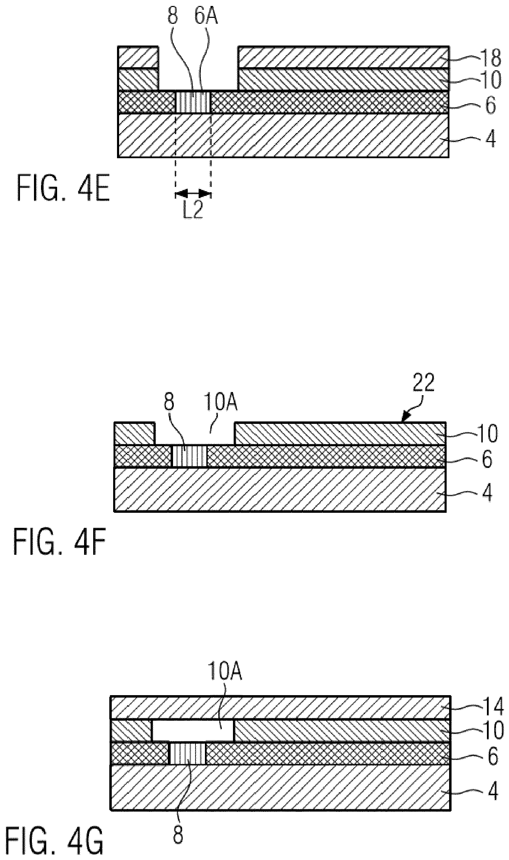

As will be explained further below with reference to Figure 4E, the reagent 8 is deposited in a groove of width L2 of the biocompatible layer 6. The reagent 8 is deposited by piezoelectric deposition, by screen printing, by flat screen printing, by rotary screen printing, by inkjet printing or by offset printing. The reagent 8 can thus be deposited by means of known and controlled techniques.

The groove of width L2 of the biocompatible layer 6 in accordance with the first embodiment is a through groove. Thus, in the first embodiment, the reagent 8 is deposited directly on the base layer 4.

According to the first embodiment, the width L2 of the groove of the biocompatible layer 6 is less than the width L1 of the channel 12 formed in the self-adhesive layer 10.

The channel 12 provides the space necessary for the fluid to be analysed to move and be in contact with the reagent 8, in particular in order to cause a chemical reaction.

The present invention also relates to a biosensor (not shown) comprising at least one transducer and a multilayer structure 2 in accordance with the first embodiment.

Figure 3 illustrates a multilayer structure 102 according to a second embodiment of the present invention.

As in the first embodiment, the multilayer structure 102 is a structure for an optical biosensor and has no electrode and/or no metal layer. The multilayer structure 102 comprises a base layer 104 and a biocompatible layer 106, comprising a reagent 108, on the base layer 104. The reagent 108 is an electrochemical or fluorescent substance deposited by piezoelectric deposition, by screen printing, by flat screen printing, by rotary screen printing, by inkjet printing or by offset printing. The reagent 108 can thus be deposited by means of known and controlled techniques.

The multilayer structure 102 furthermore comprises a pressure-sensitive self-adhesive layer 110 on the biocompatible layer 106, so that the reagent 108 is aligned with a channel 112 formed in the self-adhesive layer 110.

The multilayer structure 102 also comprises a top layer 114 on the self-adhesive layer 110.

In accordance with the present invention, the biocompatible layer 106 is deposited directly on the base layer 104 and is adhesive.

Unlike the structure 2 in the first embodiment, in the second embodiment, the groove of width L2 of the biocompatible layer 106 is not a through groove. Thus, the reagent 108 is not in contact with the base layer 104, and is deposited on the biocompatible layer 106.

In addition, in accordance with the second embodiment, the width L2 of the groove of the biocompatible layer 106 is equal to the width L1 of the channel 112 formed in the self-adhesive layer 110.

The channel 112 provides the space necessary for the fluid to be analysed to move and be in contact with the reagent 108, in particular in order to cause a chemical reaction.

The present invention also relates to a biosensor (not shown) comprising at least one transducer and a multilayer structure 102 in accordance with the second embodiment.

The base layers 4, 104 and the top layers 14, 114 may be produced from polyester, PET, polypropylene, epoxy glass, polyimide and/or paper.

The biocompatible layers 6, 106 and the self-adhesive layers 10, 110 may be produced from acrylic ester, polyacrylic ester, polyurethane acrylic ester, polyester and/or polypropylene.

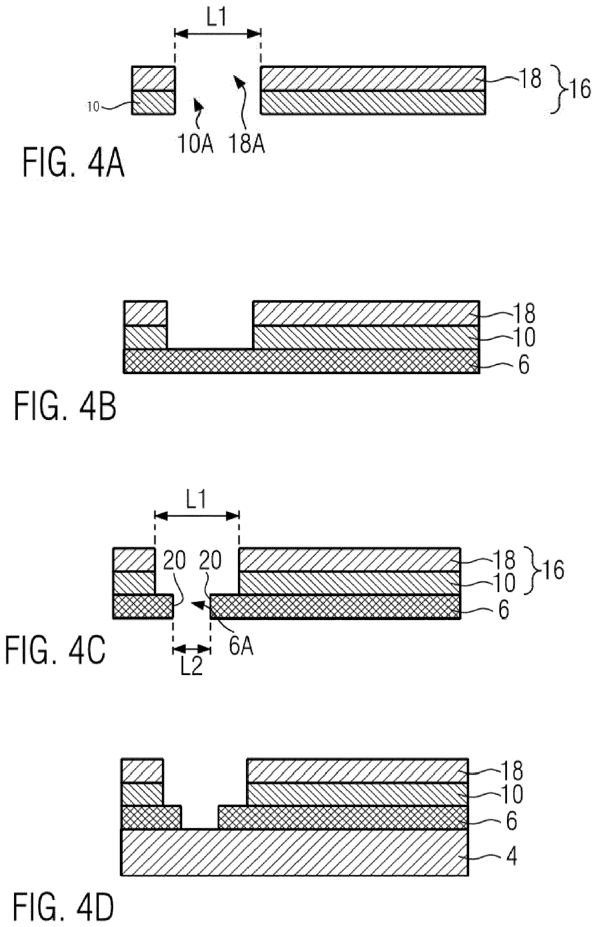

Figures 4A to 4G illustrate different steps of a method for the manufacture of the multilayer structure 2 in accordance with the first embodiment of the present invention.

The elements with the same reference numerals already used for the description of Figure 2 will not be described again in detail; reference should be made to their descriptions above.

According to the method of the first embodiment, in the first step represented by Figure 4A, a substrate layer 16 is formed by the self-adhesive layer 10 onto which a release film 18 is deposited. The release film 18 prevents the sticky surface of the self-adhesive layer 10 from adhering prematurely, because it has been covered.

During the first step, the substrate layer 16 is perforated so as to form a through opening 18A of width L1 through the release film 18 and a through opening 10A of the same width L1 through the self-adhesive layer 10.

During a second step of the method as illustrated in Figure 4B, the substrate layer 16 is deposited on the biocompatible and adhesive layer 6 by lamination, so that the self-adhesive layer 110 is deposited directly onto the biocompatible and adhesive layer 6.

Since the self-adhesive layer 10 is pressure-sensitive, no solvent, water or heat is required to activate the adhesive properties of the layer 10. During a third step of the method as illustrated in Figure 4C, a through-groove 6A of width L2 is formed in the biocompatible layer 6 by exposure and chemical etching. The irradiation consists of exposing only the zone of width L2 to ultraviolet radiation by means of a masking system.

This chemical etching step can be used to obtain a groove 6A with smooth walls 20, which provides good hydrophilic properties for the fluid to be analysed. The chemical etch may be carried out by means of a solution based on concentrated sulphuric acid, or on concentrated chloric acid or on concentrated sodium hydroxide or on butyl derivatives of the butyl ether type. Because the groove 6A is etched with a width L2 which is smaller than the width L1 of the substrate layer 16, this makes it possible to have no adhesive in the groove 6 A after the lamination step.

During a fourth step of the method as illustrated in Figure 4D, the biocompatible and adhesive layer 6 is deposited directly onto the base layer 4 by lamination.

Since there are no motifs on this base layer 4 in accordance with the present invention, lamination does not require a plurality of lamination steps. In fact, because of the adhesive properties of the biocompatible layer 6 according to the present invention, there is no need for an adhesive film or a release film for lamination of the base layer 4.

In a fifth step of the method as illustrated in Figure 4E, the reagent 8 is deposited in the groove 6A of width L2 of the biocompatible layer 6 by piezoelectric deposition, by screen printing, by flat screen printing, by rotary screen printing, by ink jet printing or by offset printing, then the reagent 8 is dried. Since the groove 6A of width L2 of the biocompatible layer 6 in accordance with the first embodiment is a through groove, the reagent 8 is deposited directly on the base layer 4.

During a sixth step of the method as illustrated in Figure 4F, the release film 18 was removed from the surface 22 of the self-adhesive layer 10.

During the seventh and last step, illustrated in Figure 4G, a top layer 14 is deposited onto the self-adhesive layer 10 by lamination in order to close the opening 10A which forms the channel 12 in which the fluid to be analysed can move. Since there are no motifs on this top layer 14 in accordance with the present invention, lamination does not require a plurality of lamination steps.

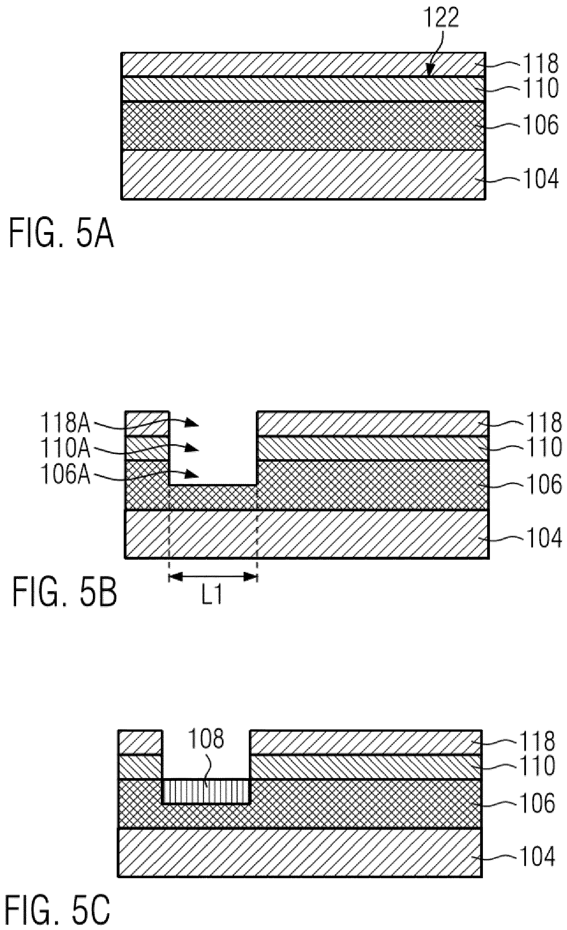

The method in accordance with the first embodiment of the invention means that the number of steps required can be reduced compared with the known prior art method and advantageously makes it possible to avoid contact between the reagent 8 and the adhesive 10, thus reducing the risk of polluting the reagent 8. Figures 5A to 5E illustrate different steps of a method for manufacturing the multilayer structure 102 in accordance with the second embodiment of the present invention.

The elements with the same reference numerals already used for the description of Figure 3 will not be described again in detail; reference should be made to their descriptions above.

In accordance with the method of the second embodiment, in the first step represented by Figures 5A, the base layer 104, for example made of PET, is used as a substrate. The biocompatible and adhesive layer 106 is deposited directly onto the base layer 104. The self-adhesive layer 110 is deposited directly onto the biocompatible layer 106. A release film 118 is deposited onto a surface 122 of the self-adhesive layer 110.

During a second step of the method as illustrated in Figure 5B, a through hole 110A, 118A is respectively formed through the self-adhesive layer 110 and the release film 118, while a blind hole 106A is formed in the biocompatible layer 106. This perforation step may be carried out by a laser. The laser is therefore configured to pierce through the self-adhesive layer 110 and the release film 118 and to remove only a portion of the biocompatible layer 106. Thus, the laser does not reach the base layer 104.

Alternatively, if the self-adhesive layer 110 is capable of being etched, the through holes 110A, 118A and the blind hole 106A may be formed by etching.

According to the second embodiment, the through holes 110A, 118A and the blind hole 106A have the same width L1.

In a variation of the second embodiment (not shown), the width of the through holes 110A, 118A may be greater than the width of the blind hole 106A. During a third step of the method as illustrated in Figure 5C, the reagent 108 is deposited in the blind hole 106A of width L1 of the biocompatible layer 106 by piezoelectric deposition, by screen printing, by flat screen printing, by rotary screen printing, by ink jet printing or by offset printing, then the reagent 108 is dried.

In the second embodiment, the reagent 108 is deposited in the blind hole 106A, i.e. in a nonthrough hole 106A. Thus, the reagent 108, unlike the first embodiment, is not in contact with the base layer 104.

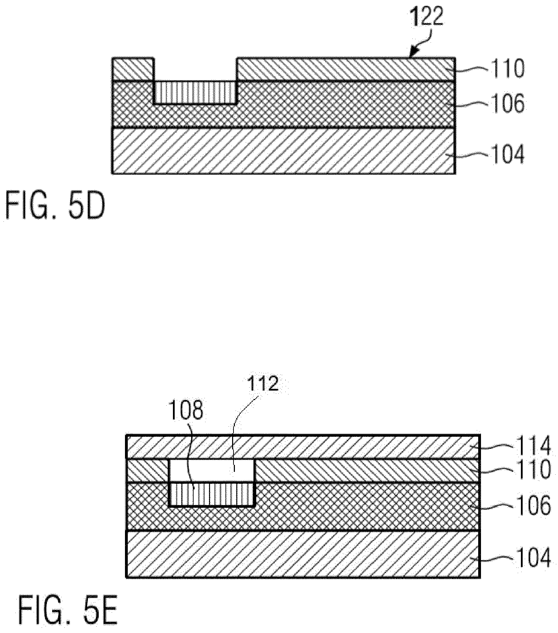

During a fourth step of the method as illustrated in Figure 5D, the release film 118 has been removed from the surface 122 of the self-adhesive layer 110.

In the fifth and final step, illustrated in Figure 5E, a top layer 114 is deposited onto the self-adhesive layer 110 by lamination in order to close the hole 110A which forms the channel 112 in which the fluid to be analysed can move. Because there are no motifs on this top layer 114 in accordance with the present invention, lamination does not require a plurality of lamination steps.

The method in accordance with the second embodiment of the invention means that a bottom of the channel 112 can consist of the biocompatible layer 106, more precisely in the blind hole 106A of the biocompatible layer 106, into which the reagent 108 is deposited. The reagent 108 is therefore not in contact with the base layer 104 in accordance with the second embodiment. It is thus not necessary for the base layer 104 to exhibit hydrophilic properties. For this reason, it is possible to use a low-cost PET base layer 104 with no hydrophilic properties.

The embodiments described are merely possible configurations, and it should be borne in mind that the individual characteristics of the various embodiments may be combined or be provided independently of one another.

## Claims

1. A multilayer structure for a biosensor, comprising:
a base layer (4, 104), and
a biocompatible layer (6, 106), comprising a reagent (8, 108), on the base layer (4, 104), and
a self-adhesive layer (10, 110) on the biocompatible layer (6, 106),
such that the reagent (8, 108) is at least partially aligned with a channel (12, 112) formed in the self-adhesive layer (10, 110), and
a top layer (14, 114) on the self-adhesive layer (10, 110),
the biocompatible layer (6, 106) being deposited directly onto the base layer (4, 104) and being adhesive.

2. The multilayer structure for a biosensor as claimed in claim 1, in which the reagent (8, 108) is an electrochemical or fluorescent substance.

3. The multilayer structure for a biosensor as claimed in claim 1 or claim 2, wherein the reagent (8, 108) is deposited in a groove (6A, 106A) of the biocompatible layer (6, 106).

4. The multilayer structure for a biosensor as claimed in claim 3, wherein the width (L2) of said groove (6A) of the biocompatible layer (6) is smaller than the width (L1) of the channel (12) of the self-adhesive layer (10).

5. The multilayer structure for a biosensor as claimed in claim 3 or 4, wherein said groove (6A) of the biocompatible layer (6) is a through groove.

6. The multilayer structure for a biosensor as claimed in one of claims 1 to 5, in which the self-adhesive layer (10, 110) is a pressure-sensitive self-adhesive layer (10, 110).

7. A biosensor comprising at least one transducer and a multilayer structure (2, 102) as claimed in one of claims 1 to 6.

8. A method for the manufacture of a multilayer structure for a biosensor, comprising the steps of:
- providing a base layer (4, 104),
- providing a biocompatible and adhesive layer (6, 106), which comprises a reagent (8, 108), directly on the base layer (4, 104),
- providing a self-adhesive layer (10, 110) comprising a channel (12, 112) in which a fluid can move on or over the biocompatible layer (6, 106), in a manner such that the reagent (8, 108) of the biocompatible layer (6, 106) is at least partially aligned with the channel (12, 112) of the self-adhesive layer (10, 110),
- providing a top layer (14, 114) deposited on the self-adhesive layer (10, 110).

9. The manufacturing method as claimed in claim 8, wherein the reagent (8, 108) is an electrochemical or fluorescent substance.

10. The manufacturing method as claimed in claim 8 or claim 9, comprising:
- a first step during which a through opening (10A) of width L1 is formed through the pressure-sensitive adhesive layer (10) and a release film (18) deposited on a surface (22) of the self-adhesive layer (10), and
- a second step during which the self-adhesive layer (10) is deposited directly onto the biocompatible and adhesive layer (6), in particular by lamination, and
- a third step for forming a groove (12) of width L2 by etching into the biocompatible and adhesive layer (6), the width L2 being smaller than the width L1, and
- a fourth step during which the biocompatible and adhesive layer (6) is deposited directly onto the base layer (4), in particular by lamination, and
- a fifth step during which the reagent (8) is deposited in the groove (12) of width L2, and
- a sixth step during which the release film (18) is removed from the self-adhesive layer (10), and
- a seventh step during which a top layer (14) is deposited onto the self-adhesive layer (10), in particular by lamination.

11. The manufacturing method as claimed in claim 8 or claim 9, comprising:
- a first step during which a release film (118) is deposited onto a surface (122) of the self-adhesive layer (110), which layer (110) is in turn deposited on the biocompatible and adhesive layer (106), which is deposited on the base layer (104),
- a second step for perforation to form a through hole (110A, 118A) through the self-adhesive layer (110) and the release film (118) and forming a blind hole (106A) in the biocompatible and adhesive layer (106),
- a third step during which the reagent (108) is deposited in the blind hole (106A) of the biocompatible and adhesive layer (106),
- a fourth step during which the release film (118) is removed from the self-adhesive layer (110), and
- a fifth step during which a top layer (114) is deposited on the self-adhesive layer (110), in particular by lamination.

12. The manufacturing method as claimed in claim 11, wherein the perforation in the second step is carried out by laser or by chemical etching.
